(19) Europäisches Patentamt
European Patent Office
Office européen des brevets

(11) Veröffentlichungsnummer: **0 082 982**
**B1**

(12) # EUROPÄISCHE PATENTSCHRIFT

(45) Veröffentlichungstag der Patentschrift: **06.08.86**

(51) Int. Cl.⁴: **C 07 D 233/60,** C 10 L 1/00, C 10 M 133/46 // (C10M133/46, 101:02)

(21) Anmeldenummer: **82111158.0**

(22) Anmeldetag: **02.12.82**

(54) **Ammoniumsalze von Halbestern des N-Hydroxiethylimidazols, deren Herstellung und Verwendung als öllösliche Korrosionsinhibitoren.**

(30) Priorität: **15.12.81 DE 3149570**

(43) Veröffentlichungstag der Anmeldung: **06.07.83 Patentblatt 83/27**

(45) Bekanntmachung des Hinweises auf die Patenterteilung: **06.08.86 Patentblatt 86/32**

(84) Benannte Vertragsstaaten: **BE DE FR GB IT NL**

(56) Entgegenhaltungen: **Keine Entgegenhaltungen**

(73) Patentinhaber: **BASF Aktiengesellschaft, Carl-Bosch-Strasse 38, D-6700 Ludwigshafen (DE)**

(72) Erfinder: **Baur, Richard, Dr., Schillerstrasse 10, D-6701 Dannstadt-Schauernheim (DE)**
Erfinder: **Oppenlaender, Knut, Dr., Otto-Dill-Strasse 23, D-6700 Ludwigshafen (DE)**
Erfinder: **Strickler, Rainer, Dr., Schroederstrasse 14, D-6900 Heidelberg (DE)**
Erfinder: **Vogel, Hans-Henning, Dr., Hans-Purrmann-Strasse 7c, D-6710 Frankenthal (DE)**
Erfinder: **Schwartz, Erich, Dr., Mohnstrasse 37, D-6700 Ludwigshafen (DE)**

EP 0 082 982 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft Ammoniumsalze von Alkylaminen und Halbestern des N-Hydroxiethylimidazols mit Phthalsäure bzw. Alkenylbernsteinsäuren, deren Herstellung und die Verwendung als Korrosionsinhibitoren in Kohlenwasserstoffen.

Auf zahlreichen Anwendungsgebieten, bei denen Metalle mit Wasser, aber auch mit Öl-Wasser-Zweiphasensystemen, wie z.B. wässrigen Emulsionen oder anderen Stoffsystemen, die Wasser enthalten, in Kontakt stehen, besteht die Gefahr der Korrosion. Es ist bekannt, dass auch durch Flüssigkeiten, die nur geringe Mengen Wasser enthalten, wie Otto- und Dieselkraftstoffe, Korrosion verursacht werden kann: Kondenswasser sammelt sich in Tropfenform am Boden von Kraftstofftanks oder in Rohrleitungen und wirkt zusammen mit aus der Luft aufgenommenem Sauerstoff korrosiv, was zu Schäden an Blech- und Bleischicht von Tanks, aber auch am ganzen Kraftstoffleitungssystem von Motoren führen kann.

In der Regel ist man bemüht, die Korrosionswirkung einer Flüssigkeit gegenüber Maschinen- und Apparateteilen, Behältern und Rohrwandungen und sonstigen metallischen Konstruktionselementen durch Korrosionsinhibitoren zu verhindern oder aufzuheben. Zur Verhinderung der Korrosion des Eisens werden normalerweise in nicht wässrigen Flüssigkeiten langkettige, hochmolekulare organische Säuren, Ester oder Amine mit oberflächenaktiven Eigenschaften eingesetzt. Als Korrosionsschutz-Additive für Buntmetalle sind aus DE-AS 2 265 088 Benzo- und Tolyltriazol bekannt.

Benzo- und Tolyltriazol sind zwar universell einsetzbare Korrosionsinhibitoren für Buntmetalle, aber ihre aufwendige Synthese bringt hohe Kosten mit sich, und durch ihren relativ polaren Charakter ist ihre Löslichkeit in Medien, die im wesentlichen nur aliphatische Kohlenwasserstoffe enthalten, begrenzt.

Das Ziel der Erfindung bestand daher in der Bereitstellung öllöslicher Korrosionsinhibitoren, die ein möglichst breites Wirkungs- und Anwendungsspektrum besitzen und die oben genannten Nachteile nicht aufweisen.

Überraschenderweise wurde nun gefunden, dass neue Verbindungen der Formel

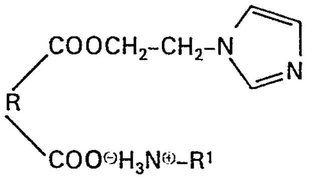

I,

in der R den Rest der o-Phthalsäure oder einer Alkenylbernsteinsäure mit einer Alkenylseitenkette von 8 bis 20 Kohlenstoffatomen und $R^1$ einen Alkylrest mit 8 bis 13 Kohlenstoffatomen bedeutet, korrosionsschützend auf Buntmetalle und deren Legierungen, insbesondere Blei und Zink, wirken.

Die Ammoniumsalze der Halbester der Formel I erhält man durch Umsetzung von Phthalsäureanhydrid oder von Alkenylbernsteinsäureanhydriden mit N-Hydroxiethylimidazol zum Halbester und anschliessende Neutralisation mit Monoaminen mit 8 bis 13 Kohlenstoffatomen.

Die Herstellung der Alkenylbernsteinsäureanhydride erfolgt in an sich bekannter Weise durch thermische En-Addition von Maleinsäureanhydrid an die entsprechenden α-Olefine mit 8 bis 20 Kohlenstoffatomen.

Die Addition erfolgt in der aus Chemiker Zeitung 100, Nr. 5, S. 203–211 (1976) bekannten Weise bei 180–220°C unter Normaldruck oder unter Druck in Substanz oder durch Zugabe von Lösungsmitteln.

N-Hydroxiethylimidazol ist gemäss DE-PS 854 955 durch Umsetzung von Imidazol mit Ethylenoxid oder gemäss US-PS 3 178 446 durch Umsetzung von Imidazol mit Ethylencarbonat erhältlich.

Die erfindungsgemässen Ammoniumsalze der Halbester erhält man durch Umsetzung im Molverhältnis von ungefähr 1:1 von Alkenylbernsteinsäureanhydriden oder Phthalsäureanhydrid mit N-Hydroxiethylimidazol in Substanz oder in unter den Reaktionsbedingungen inerten Lösungsmitteln, wie Aceton, Methylethylketon, Tetrahydrofuran, Dioxan, Dimethylformamid, Essigsäureethylester, Toluol, Xylol usw., bei Temperaturen zwischen 40 und 100°C, bevorzugt zwischen 60–80°C, und anschliessende Neutralisation mit einem Monoamin mit 8 bis13 Kohlenstoffatomen bei 60–80°C in Gegenwart oder Abwesenheit von Katalysatoren. Als Monoamine mit 8 bis 13 Kohlenstoffatomen kommen beispielsweise Ethylhexylamin und Tridecylamin in Betracht.

Die Umsetzung zum Ammoniumsalz kann durch Titration der Säurezahl und IR-spektroskopisch verfolgt werden. Als α-Olefine zur Herstellung der Alkenylbernsteinsäureanhydride kommen beispielsweise Diisobuten, 1,2-Dodecen, Trimerbuten, Tetramerpropen, Pentapropylen, Hexapropylen, Tetrabuten, Pentabuten, aber auch α-Olefin-Gemische wie $C_{16/18}$-α-Olefin oder $C_{16/18/20}$-α-Olefine in Betracht.

Besonders bevorzugte Dicarbonsäureanhydride sind Diisobutenyl-bernsteinsäureanhydrid, Pentapropenyl-bernsteinsäureanhydrid, $C_{16/18}$-α-Olefin/MSA-Addukt und Phthalsäureanhydrid.

Die erfindungsgemässen Ammoniumsalze der Halbester sind für eine Vielzahl von Anwendungszwecken als Korrosionsinhibitoren, insbesondere in Kohlenwasserstoffen, wirksam.

Die Anwendungszwecke schliessen technische Wärmeübertragungssysteme, Pumpenrohre und andere Metalleinrichtungen für Öl-Förderanlagen und Pipelines, insbesondere Kraftstoffleitungen von Benzin- und Dieselmotoren sowie alle Motorteile, wie Vergaser, Einspritzpumpe, Kolben usw., aber auch Tanks zur Lagerung und zum Transport von Kraftstoffen mit ein. Als besonders korrosionsgefährdet gelten dabei die Vergaserbauteile (bestehend aus Zinkspritzguss = 95% Zink, 4% Aluminium und 1% Kupfer) sowie die verbleiten Teile von Kraftstofftanks.

Der Korrosionsinhibitor kann dabei direkt in Konzentrationen von 0,1 bis 1000 ppm, bevorzugt von 1 bis 500 ppm, dem Kraftstoff zugesetzt werden. Weiterhin möglich ist auch die Zugabe des Inhibitors im Rahmen eines handelsüblichen Kraftstoff-Additiv-Gemisches, bestehend aus einem Ventil- und Vergaserreiniger, einem Oxidationsinhibitor, einem filmbildenden Korrosionsschutzadditiv (zur Verhinderung der Korrosion des Eisens) usw.

Beispiele

A) Herstellung des N-Hydroxiethylimidazols

884 g (13 Mol) Imidazol und 44,2 g Wasser werden im Rührautoklaven bei 90–100°C und max. 10 bar Druck mit 572 g (13 Mol) Ethylenoxid umgesetzt. Nach weiterem Rühren bis zur Druckkonstanz wird bei 90°C und Wasserstrahlvakuum (15 mm Hg) entwässert.
Ausbeute: 1446 g (99% d. Theorie)

B) Herstellung von $C_{16/18}$-$\alpha$-Olefin/Maleinsäureanhydrid-Addukt

Eine Mischung aus 714 g (3 Mol) $C_{16/18}$-$\alpha$-Olefin wird mit 294 g (3 Mol) Maleinsäureanhydrid unter Stickstoff langsam auf 210°C erhitzt und anschliessend noch 8 Std. auf diese Temperatur erhitzt.

Nicht umgesetztes Maleinsäureanhydrid wird am Wasserstrahlvakuum bis zu einer Sumpftemperatur von 155°C abdestilliert.
Ausbeute: 990,5 g (98% d. Theorie)
SZ: 170 mg KOH/g Theorie 167

Beispiel 1

a) Eine Mischung aus 268,8 g (0,8 Mol) $C_{16/18}$-$\alpha$-Olefin/Maleinsäureanhydrid-Addukt und 89,7 g (0,8 Mol) N-Hydroxiethylimidazol wird 4 Stunden bei 80°C gerührt. Man erhält 350 g Halbester (98% d. Theorie) mit SZ 107,5 (Theorie 125,2). Nach Zugabe von 500 ml Toluol wird bei Raumtemperatur langsam mit 133,7 g (0,67 Mol) Tridecylamin neutralisiert. Man rührt 2 Stunden bei 60°C nach und entfernt anschliessend das Toluol am Wasserstrahlvakuum bis 60°C.
Ausbeute: 483 g (100% d. Theorie) Tridecylammoniumsalz des Halbesters aus $C_{16/18}$-$\alpha$-Olefin/Maleinsäureanhydrid-Addukt und N-Hydroxiethylimidazol.

b) Korrosionstest

Eine 250 ml-Glasflasche wird mit 100 ml Superbenzin (Erdölraffinerie Mannheim) gefüllt. Das Produkt gemäss a) wird in Mengen zwischen 10 und 1000 ppm dosiert. Dann werden 4 ml destilliertes Wasser zugegeben. Testcoupons der Grösse 50 mm×20 mm×2 mm werden geschmirgelt (Körnung 20), mit Toluol entfettet und gewogen. Die Testflaschen schüttelt man eine Minute kräftig, damit das Wasser im Benzin verteilt wird. Die Metallcoupons werden eingelegt und 14 Tage bei 20 bis 25°C gelagert. Dann werden die Coupons mit 15%iger Salzsäure, die noch 1% Propargylalkohol als Beizinhibitor enthält, gereinigt, entfettet und getrocknet. Die Bestimmung des Gewichtsverlustes erfolgt durch Wiegen (Angaben in ‰ Gewichtsverlust). In der Regel werden Mehrfachbestimmungen durchgeführt.

| Dosierung | Metall | Blei | Zinkspritzguss 95% Zn, 4% Al, 1% Cu |
|---|---|---|---|
| ohne Zusatz | | 1,59‰ | 4,06‰ |
| Verbindung des Beispiels 1 | 10 ppm | 1,14‰ | 1,65‰ |
| | 50 ppm | 0,67‰ | 1,06‰ |
| Additivgemisch* | 300 ppm | 0,45‰ | 1,55‰ |
| | 500 ppm | 0,48‰ | 1,27‰ |
| Additivgemisch mit 1 Gew.-% der Verbindung gemäss Beispiel 1 | 300 ppm | 0,33‰ | 1,33‰ |
| | 500 ppm | 0,33‰ | 1,18‰ |

* Additiv-Gemisch eines handelsüblichen Ventil- und Vergaserreinigers ohne Benzotriazol als Korrosionsinhibitor.

Beispiel 2

a) Eine Mischung aus 105 g (0,5 Mol) Diisobutenylbernsteinsäureanhydrid (hergestellt gemäss den Angaben unter B aus 336 g Diisobuten und 294 g Maleinsäureanhydrid) und 56,1 g (0,5 Mol) N-Hydroxiethylimidazol wird 4 Stunden bei 80°C gerührt. Man erhält 160 g (99% d. Theorie) Halbester mit SZ =185,7 (Theorie 174,1). Nach Zugabe von 500 ml Toluol neutralisiert man bei Raumtemperatur langsam mit 106,3 g (0,53 Mol) Monotridecylamin. Man rührt 2 Stunden bei 60°C nach und entfernt anschliessend das Toluol am Wasserstrahlvakuum bis 60°C.
Ausbeute: 266 g Tridecylammoniumsalz des Halbesters aus Diisobuten/Maleinsäureanhydrid und N-Hydroxiethylimidazol.

b) Korrosionstest, wie in Beispiel 1b beschrieben, Angaben in ‰ Gewichtsverlust.

| Dosierung | Metall | Blei | Zinkspritzguss<br>95% Zn, 4% Al, 1% Cu |
|---|---|---|---|
| ohne Zusatz | | 0,88‰ | 2,22‰ |
| Zusatz gemäss<br>Beispiel 2 | 10 ppm<br>20 ppm<br>30 ppm<br>50 ppm | 0,53‰<br>0,53‰<br>0,37‰<br>0,30‰ | 0,48‰<br>0,50‰<br>0,41‰<br>0,36‰ |

Beispiel 3

a) Eine Mischung aus 154 g (0,5 Mol) Pentapropenylbernsteinsäureanhydrid (hergestellt gemäss den Angaben unter B aus 210 g Pentapropylen und 98 g Maleinsäureanhydrid) und 56,1 g (0,5 Mol) N-Hydroxiethylimidazol wird 4 Stunden bei 80°C gerührt. Man erhält 210 g (10% d. Theorie) Halbester mit SZ 175 (Theorie 173,3). Nach Zugabe von 200 ml Toluol neutralisiert man bei Raumtemperatur langsam mit 99,5 g (0,5 Mol) Monotridecylamin. Man rührt 2 Stunden bei 60°C nach und entfernt das Toluol am Wasserstrahlvakuum bis 60°C.

Ausbeute: 305 g (98% d. Theorie) Tridecylammoniumsalz des Halbesters aus Pentapropylen/Maleinsäureanhydrid-Addukt und N-Hydroxiethylimidazol.

b) Korrosionstest, gemäss den Angaben von Beispiel 1b, Angaben in ‰ Gewichtsverlust.

| Dosierung | Metall | Blei | Zinkspritzguss | Stahl 37 |
|---|---|---|---|---|
| ohne Zusatz | | 1,06‰ | 0,77‰ | 0,17‰ |
| Zusatz gemäss<br>Beispiel 3 | 10 ppm<br>30 ppm<br>50 ppm | 0,40‰<br>0,55‰<br>0,66‰ | 0,20‰<br>0,32‰<br>0,31‰ | 0,03‰<br>0,01‰<br>0 ‰ |

Beispiel 4

a) Eine Mischung aus 74 g (0,5 Mol) Phthalsäureanhydrid und 56,1 g (0,5 Mol) N-Hydroxiethylimidazol wird 4 Stunden bei 80°C gerührt. Man erhält 125 g (96% d. Theorie) Halbester mit SZ = 210 (Theorie 215,6). Nach Zugabe von 200 ml Toluol neutralisiert man bei Raumtemperatur langsam mit 99,5 g (0,5 Mol) Monotridecylamin. Man rührt 2 Stunden bei 60°C nach und entfernt anschliessend das Toluol am Wasserstrahlvakuum bis 60°C.

Ausbeute: 224 g (100% d. Theorie) Tridecylammoniumsalz des Halbesters aus Phthalsäureanhydrid und N-Hydroxiethylimidazol.

b) Korrosionstest, gemäss den Angaben des Beispiels 1b, Angaben in ‰ Gewichtsverlust.

| Dosierung | Metall | Blei | Zinkspritzguss |
|---|---|---|---|
| ohne Zusatz | | 1,63‰ | 1,24‰ |
| Zusatz gemäss<br>Beispiel 4 | 30 ppm<br>50 ppm<br>100 ppm | 1,32‰<br>1,07‰<br>0,93‰ | 0,52‰<br>0,70‰<br>0,55‰ |

**Patentansprüche**

1. Verbindungen der Formel

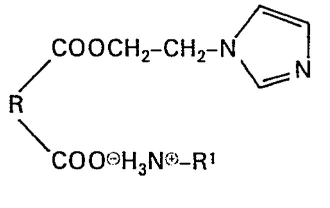

I,

in der R den Rest der o-Phthalsäure oder einer Alkenylbernsteinsäure mit einer Alkenylseitenkette von 8 bis 20 Kohlenstoffatomen und $R^1$ einen Alkylrest mit 8 bis 13 Kohlenstoffatomen bedeutet.

2. Verfahren zur Herstellung von Verbindungen der Formel I gemäss Anspruch 1, dadurch gekennzeichnet, dass man Phthalsäureanhydrid bzw. Alkenylbernsteinsäureanhydrid mit molaren Mengen 1-Hydroxiethylimidazol verestert und den erhaltenen Monoester mit einem Amin $R^1$-$NH_2$, in der $R^1$ einen Alkylrest mit 8 bis 13 Kohlenstoffatomen bedeutet, zum Ammoniumsalz umsetzt.

3. Verwendung von Verbindungen der Formel I gemäss Anspruch 1 als korrosionsinhibierender Zusatz in Mineralölen.

4. Treibstoffe für Ottomotoren und Dieselmotoren, enthaltend als Korrosionsinhibitoren geringe Mengen von Verbindungen der Formel

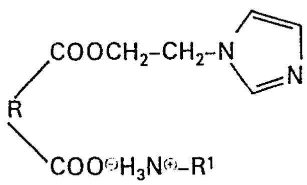

I,

in der R den Rest der o-Phthalsäure oder einer Alkenylbernsteinsäure mit einer Alkenylseitenkette von 8 bis 20 Kohlenstoffatomen und $R^1$ einen Alkylrest mit 8 bis 13 Kohlenstoffatomen bedeutet.

5. Treibstoff für Otto- und Dieselmotoren gemäss Anspruch 4, enthaltend 0,1 bis 1000 ppm der Verbindungen der Formel I.

**Claims**

1. A compound of the formula

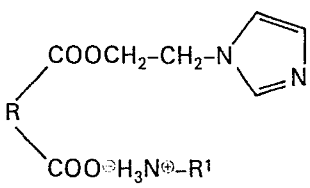

I,

where R is the remaining part of o-phthalic acid or an alkenyl succinic acid having an alkenyl side chain of 8 to 20 carbon atoms, and $R^1$ is alkyl of 8 to 13 carbon atoms.

2. A process for the preparation of a compound of the formula I as claimed in claim 1, wherein phthalic anhydride or alkenyl succinic anhydride is esterified with molar amounts of 1-hydroxyethylimidazole, and the resulting monoester is reacted with an amine $R^1$-$NH_2$, where $R^1$ is alkyl of 8 to 13 carbon atoms, to give the ammonium salt.

3. The use of a compound of the formula I as claimed in claim 1 as corrosion-inhibiting additive in mineral oils.

4. Fuels for Otto and diesel engines, containing, as corrosion inhibitor, a small amount of a compound of the formula

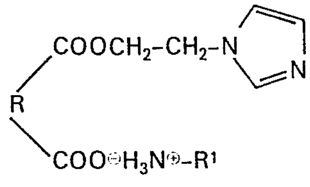

I,

where R is the remaining part of o-phthalic acid or an alkenyl succinic acid having an alkenyl side chain of 8 to 20 carbon atoms, and $R^1$ is alkyl of 8 to 13 carbon atoms.

5. Fuels for Otto and diesel engines, as claimed in claim 4 and containing from 0.1 to 1000 ppm of a compound of the formula I.

**Revendications**

1. Composés de la formule

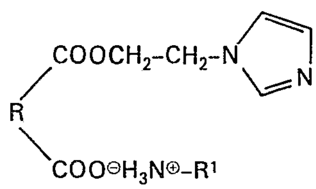

I,

dans laquelle R désigne le reste de l'acide orthophtalique ou d'un acide alcényl-succinique avec une chaîne alcényle latérale en $C_8$ à $C_{20}$ et $R^1$ un radical alkyle en $C_8$ à $C_{13}$.

2. Procédé de préparation de composés de la formule I selon la revendication 1, caractérisé en ce que l'on procède à une estérification de l'anhydride phtalique ou d'un anhydride alcényl-succinique par une proportion molaire de 1 (lire: N)-hydroxyéthyl-imidazole, puis transforme le mono-ester obtenu par réaction avec une amine $R^1$-$NH_2$, où $R^1$ représente un radical alkyle en $C_8$ à $C_{13}$, en sel d'ammonium.

3. Utilisation de composés de la formule I selon la revendication 1 comme additifs inhibant la corrosion dans des huiles minérales.

4. Carburants pour moteurs à essence et moteurs diesel, contenant en tant qu'inhibiteurs de la corrosion des proportions mineures de composés de la formule

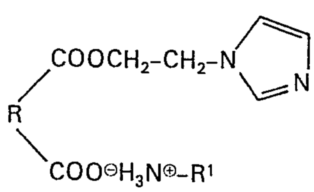

I,

dans laquelle R désigne le reste de l'acide orthophtalique ou d'un acide alcényl-succinique avec une chaîne alcényle latérale en $C_8$ à $C_{20}$ et $R^1$ un radical alkyle en $C_8$ à $C_{13}$.

5. Carburants pour moteurs à essence et moteurs diesel, selon la revendication 4, contenant entre 0,1 et 1000 ppm de composés de la formule I.